# EUROPÄISCHE PATENTSCHRIFT

(11) **EP 1 870 022 B1**
(45) Veröffentlichungstag und Bekanntmachung des Hinweises auf die Patenterteilung: **11.05.2016**
(21) Anmeldenummer: 07011078.8
(22) Anmeldetag: 06.06.2007
(51) Int. Cl.: A61B 1/24, A61C 1/08

(54) **Medizinischer Handgriff mit Beleuchtungsvorrichtung und Verfahren zur Herstellung**
Medical handle with a lighting device and method of manufacturing
Poignée medical avec dispositif d'eclairage et procédé de fabrication

(30) Priorität: 22.06.2006 EP 06012842
(43) Veröffentlichungstag der Anmeldung: 26.12.2007
(73) Patentinhaber: W & H Dentalwerk Bürmoos GmbH, 5111 Bürmoos (AT)
(72) Erfinder: Brandstätter, Andreas, 5120 St. Pantaleon 208 (AT); Schalda, Anton, 5121 Ostermiething (AT); Wendtner, Wolfgang, 5121 Lamprechtshausen (AT)

(56) Entgegenhaltungen:
- EP-A2- 1 093 765
- WO-A-2004/107457
- DE-A1- 10 359 501
- US-A- 3 109 238
- US-A- 6 137 224

## Beschreibung

Die vorliegende Erfindung betrifft einen medizinischen Handgriff mit einer Beleuchtungsvorrichtung mit zumindest einem optischen Halbleiterbauelement als Lichtquelle und ein Verfahren zur Herstellung eines derartigen Handgriffs.

Ein medizinischer Handgriff mit einer Beleuchtungsvorrichtung mit einer Leuchtdiode (LED) ist aus dem Dokument EP 1.093.765 A2 bekannt. Die Beleuchtungsvorrichtung besteht aus einem Halter mit einem U-förmigen Querschnitt in dem die LEDs aufgenommen und mit einem transparenten, wärmebeständigen Kunstharz vergossen werden. Das ursprünglich flüssige Kunstharz durchläuft nach dem Eingießen in den Halter eine chemische Reaktion, wodurch es aushärtet, so dass die LEDs von der Umgebung abgedichtet sind. Die Lichtabgabe und die Lichtleitung erfolgen durch das ausgehärtete Kunstharz. Durch diesen Aufbau soll erreicht werden, dass die Beleuchtungsvorrichtung in einem Autoklaven sterilisierbar ist

Die Beleuchtungsvorrichtung dieses medizinischen Handgriffs sowie die Herstellung eines medizinischen Handgriffs mit einer derartigen Beleuchtungsvorrichtung weisen jedoch mehrere Nachteile auf. So ist insbesondere die Notwendigkeit, für jede Beleuchtungsvorrichtung einen eigenen Halter zur Verfügung stellen zu müssen, in den das Kunstharz eingegossen wird, für die Herstellung der Beleuchtungsvorrichtung hinderlich und erhöht den Hetstellungsaufwand und die Lager- und Herstellungskosten. Der Halter ist jedoch unbedingt notwendig, da die chemische Aushärtereaktion des Kunstharzes nur langsam abläuft und der Halter während der flüssigen Phase des Kunstharzes bis zu dessen endgültiger Aushärtung zur Formgebung und Lagerung des Kunstharzes und zur Aufnahme der LEDs dient. Da das Kunstharz an dem Halter anhaftet, kann dieser nicht oder nur unter beträchtlichem Aufwand, zum Beispiel durch vorheriges Anbringen einer Beschichtung an den Wänden des Halters, von der ausgehärteten Kunstharzmasse gelöst werden, so dass er meist mit der Beleuchtungsvorrichtung oder als Teil davon verbaut wird.

Die Schrift DE 103 59 501 offenbart ein dentales Funktionshandstück mit einem Lichtabgabeelement, das einen Abstrahlkörper mit Medienleitungen für Luft und Wasser und eine im Abstrahlkö per aufgenommene LED umfasst.

Die Patentschrift US 3,109,238 beschreibt dentale, schnurlose Hand- und Winkelstücke mit einer integrierten Energiequelle und einer im Bereich des Bohrers angeordneten Beleuchtungsvorrichtung mit Glühbirnen.

Die Patentschrift US 6,137,224 offenbart ein alphanummerisches Display mit einem Substrat und LEDs. Die LEDs werden durch das punktförmig oder kleinflächig auf dem Substrat aufgetragene Verkapselungsmaterial, zum Beispiel einem thermoplastischen Kunststoff, umschlossen und auf dem Substrat mechanisch befestigt.

Aus dem Dokument WO 2004/107457 A2 sind Beleuchtungsvorrichtungen mit LEDs für Taschenlampen bekannt, wobei die LEDs mit Epoxyharz oder alternativen Materialien, u.a. thermoplastischem Kunststoff, gekapselt sind.

Der vorliegenden Erfindung liegt daher die Aufgabe zugrunde ein Verfahren zur Herstellung eines medizinischen Handgriffs mit einer Beleuchtungsvorrichtung und einen medizinischen Handgriff mit einer Beleuchtungsvorrichtung zu schaffen, der die oben genannten Nachteile nicht aufweist. Der Handgriff soll eine Beleuchtungsvorrichtung aufwelsen, die einfacher und kostengünstiger herstellbar ist, d.h. dass insbesondere nicht jeder Beleuchtuhgsvorrichtung ein eigener Halter zur Verfügung gestellt werden muss, in den die optischen Halbleiterbauelemente oder LEDs sowie die sie umhüllende Füllmasse einzubringen sind. Der Handgriff und die Beleuchtungsvorrichtung sollen des Weiteren sterilisierbar sein und es sollen die Lichttransmission durch die Beleuchtungsvorrichtung und die Lichtausbeute auch nach oftmaliger Sterilisation möglichst gleich bleibend sein.

Diese Aufgabe wird gemäß der vorliegenden Erfindung durch ein Verfahren zur Herstellung eines Handgriffs mit den Merkmalen des Anspruchs 1 und einen Handgriff mit den Merkmalen des Anspruchs 8 gelöst. Besonders vorteilhafte Ausführungsformen sind in den Unteransprüchen angeführt.

Durch die Umhüllung oder Kapselung der Beleuchtungsvorrichtung mit dem zumindest einen optischen Halbleiterbauelement mit einem transparenten, thermoplastischen Kunststoff entfällt die Notwendigkeit, für jede Beleuchtungsvorrichtung einen eigenen Halter herstellen, lagern und verbauen zu müssen: Erfolgt das Umhüllen des zumindest einen optischen Halbleiterbauelements, wie weiter unten noch genauer beschrieben, zum Beispiel mit flüssigem thermoplastischen Kunststoff, der in eine Form eingefüllt wird, in der sich das optische Halbleiterbauelement befindet, so läuft der Aushärtevorgang des thermoplastischen Kunststoffs um ein vielfaches schneller ab als mit Kunstharz, da der Aushärtevorgang nicht durch eine chemische Reaktion sondern durch einen Abkühlvorgang bewirkt wird. Das Aushärten des thermoplastischen Kunststoffs findet somit in, insbesondere auch in Hinblick auf produktionstechnische und wirtschaftliche Vorgaben, akzeptablen und derart kurzen Zeiträumen statt, dass zumindest ein ausreichendes Fortschreiten des Aushärteprozesses abgewartet und anschließend das mit dem thermoplastischen Kunststoff umhüllte zumindest eine optische Halbleiterbauelement aus der Form entfernt werden kann. Da der abgekühlte thermoplastische Kunststoff nicht oder kaum an der üblicherweise metallischen Form haftet, ist das Entfernen aus der Form problemlos durchführbar. Die Form kann im Weiteren für die Herstellung einer großen Zahl von Umhüllungen verwendet werden.

Das optische Halbleiterbauelement ist somit direkt im thermoplastischen Kunststoff angeordnet bzw. darin eingelegt, wobei der thermoplastische Kunststoff bzw. die unverbaute Beleuchtungsvorrichtung nach Beendigung des Umhüllungsverfahrens und nach dem Lösen aus der Form keinen Halter mehr aufweist, in dem der thermoplastische Kunststoff aufgenommen ist. Mit der Verwendung des thermoplastischen Kunststoffs erfüllt die Beleuchtungsvorrichtung auch Vorgaben zur elektrischen Sicherheit, insbesondere wird verhindert, dass auf den Anwender oder auf den Patient während des Betriebs oder bei Auftreten eines Defekts Strom übertragen wird.

Die Beleuchtungsvorrichtung wird primär zur Beleuchtung einer Präparationsstelle und / oder eines Werkzeugs, das durch den Handgriff angetrieben wird, mit sichtbarem Licht verwendet. Alternativ oder zusätzlich kann die Beleuchtungsvorrichtung jedoch auch für therapeutische oder diagnostische Anwendungen eingesetzt werden, zum Beispiel zum Aushärten von photosensitivem Material, insbesondere Füllmaterial für Zahnkavitäten, zum Bleichen (bleachen) von Zähnen oder zur Detektion von kariösem Zahngewebe. In diesem Fall werden optische Halbleiterbauelement oder LEDs verwendet, die eine für die jeweilige Anwendung geeignete Wellenlänge, die auch außerhalb des Spektrums von sichtbarem Licht liegen kann, abgeben.

Das durch den transparenten, thermoplastischen Kunststoff umhüllte optische Halbleiterbauelement ist bevorzugt als Halbleiter-Chip ausgebildet, der insbesondere Teil eines Beleuchtungselements ist, zum Beispiel einer Leuchtdiode (LED). Die Leuchtdiode kann wiederum weitere Bauteile umfassen, so zum Beispiel Schutzhüllen für den Halbleiter-Chip oder Konversionsmaterial zur Umwandlung der von dem zumindest einem optischen Halbleiterlement abgestrahlten Wellenlänge. Die Beleuchtungsvorrichtung ist mit zumindest einem optischen Halbleiterbauelement ausgestattet, selbstverständlich können jedoch auch mehrere, elektrisch seriell oder parallel geschaltete optische Halbleiterbauelemente vorgesehen sein.

Das Umhüllen des zumindest einen optischen Halbleiterbauelements mit einem transparenten, thermoplastischen Kunststoff umfasst den vollständigen Einschluss des optischen Halbleiterbauelements in der Kunststoffmasse. Auch kann der thermoplastische Kunststoff das zumindest eine optische Halbleiterbauelement direkt kontaktieren, als auch beabstandet davon angeordnet sein. Zum Umhüllen eignen sich verschiedene transparente, thermoplastische Kunststoffe, zum Beispiel statistische-Propylen-Copolymere (PPR), Polycarbonate (PC), Polymethylenpenten (PMP), Cyclooleofin-Copolymere (COC) oder Polyphenylsulfon (PPSU). Neben ihrer hohen Transparenz weisen diese Kunststoffe eine gute UV-Beständigkeit, eine geringe Tendenz zur Verfärbung oder Vergilbung und eine chemische Beständigkeit insbesondere gegenüber Wasserdampf und Desinfektions-, Reinigungs- und Pflegemitteln auf. Sie sind damit sowohl wiederholten Sterilisations-, Desinfektions-, Reinigungs- und Pflegebehandlungen aussetzbar, als auch für medizinische, insbesondere dental-medizinische, Anwendungen einsetzbar.

Die Befestigung der Beleuchtungsvorrichtung kann an der Außenseite des medizinischen Handgriffs erfolgen, zum Beispiel durch eine Steck- oder Klemmverbindung. Bevorzugt ist die Beleuchtungsvorrichtung im Inneren des Handgriffs angeordnet, zum Beispiel durch eine Steck- oder Klemmverbindung oder durch Verkleben, wo sie für den Anwender weniger störend ist. Besonders bevorzugt ist die Beleuchtungsvorrichtung rund um den Werkzeughalter oder das Werkzeug, zum Beispiel einen rotierenden Bohrer, eine hin- und herbewegbare Feile oder Säge oder ein dentales, in Schwingung versetzbares Zahnsteinentfernungswerkzeug angeordnet, so dass eine schattenfreie, lichtstarke Beleuchtung möglichst in der Nähe der Behandlungsstelle vorliegt. In diesem Fall ist die Beleuchtungsvorrichtung zumindest teilweise im Inneren des Handgriffs angeordnet, wobei die Oberfläche des Lichtabgabefensters gleichzeitig auch das Vorderende oder eine Seitenbegrenzung des Handgriffs bildet.

In einem Ausführungsbeispiel erfolgt das Umhüllen des zumindest einen optischen Halbleiterbauelements durch zur Verfügung stellen eines festen, bevorzugt als Granulat oder Pulver vorliegenden, thermoplastischen Kunststoffs, Verflüssigen des thermoplastischen Kunststoffs und Aushärten des thermoplastischen Kunststoffs. Der Vorteil dieses Ausführungsbeispiels besteht darin, dass beim Umhüllen des zumindest einen optischen Halbleiterbauelements außer einer Änderung des Aggregatzustands keine weiteren Veränderungen des Kunststoffes notwendig sind. Insbesondere laufen keine chemischen Reaktionen während des Aushärtens ab, wodurch zum Beispiel die Gefahr des unvollständigen Aushärtens und des damit verbundenen Entstehens von Rissen und Undichtheiten erheblich verringert ist. Damit ist auch die Dauer des Umhüllens und Aushärtens bei Verwendung von thermoplastischem Kunststoff im Vergleich zur Verwendung von Kunstharzen deutlich geringer.

Insbesondere erfolgt das Umhüllen des zumindest einen optischen Halbleiterbauelements und damit die Änderung des Aggregatzustands durch Erhitzen des thermoplastischen Kunststoffs, bevorzugt auf Temperaturen von maximal etwa 310°C, besonders bevorzugt auf Temperaturen von maximal etwa 280°C. Bei Einhalten dieser Temperaturobergrenzen wird eine Beschädigung der optischen Halbleiterbauelemente beim Umhüllen aufgrund zu hoher thermischer Belastung vermieden.

Um eine definierte Form der Kunststoffhülle des optischen Halbleiterbauelements oder der Beleuchtungsvorrichtung zu erhalten, wird in einem weiteren Ausführungsbeispiel vor dem Umhüllen des zumindest einen optischen Halbleiterbauelements mit dem thermoplastischen Kunststoff das zumindest eine optische Halbleiterbauelement in einer Form angeordnet und anschließend der thermoplastische Kunststoff in die Form eingebracht. Die Form ist dabei so ausgestaltet, dass an den Außenseiten der Umhüllung unterschiedliche Elemente gebildet werden, zum Beispiel Befestigungselement in Form von Steckteilen zur Befestigung der Beleuchtungsvorrichtung am Handgriffelement oder optische Elemente, zum Beispiel Linsen am Lichtabgabefenster. Zusätzlich oder alternativ ist die Form derart beschaffen, dass im Inneren der Kunststoffhülle Funktionselemente gebildet werden, zum Beispiel Bohrungen, Sacklochbohrungen, Aufnahmen oder Verbindungs- oder Verschlusselemente. Bevorzugt ist die Umhüllung des optischen Halbleiterbauelements ringförmig oder hohlzylindrisch geformt, so dass durch die zentrale Bohrung ein Bauelement, zum Beispiel eine Welle, ein Schaft, oder ein Werkzeug durchführbar ist.

Um eine gleichmäßige und vollständige Befüllung der Form mit dem thermoplastischen Kunststoff zu gewährleisten, wird der verflüssigte Kunststoff bevorzugt unter Druck in die Form eingespritzt.

In einem weiteren Ausführungsbeispiel wird das zumindest eine optische Halbleiterbauelement derart von dem thermoplastischen Kunststoff umhüllt, dass der thermoplastische Kunststoff entweder zumindest die Strahlung emittierende Fläche des zumindest einen optischen Halbleiterbauelements oder an die Strahlung emittierende Fläche anschließende Bauteile kontaktiert. Dadurch wird eine effektive und möglichst verlustarme Weiterleitung der emittierten Strahlung mit einer geringen Anzahl an von der Strahlung zu überwindenden Medien-oder Materialübergängen erreicht.

Die Beleuchtungsvorrichtung umfasst einen Träger für das zumindest eine optische Halbleiterbauelement, insbesondere eine Platine oder eine Multilayer-Keramik, und eine elektrische Versorgungseinrichtung zur Stromversorgung des zumindest einen optischen Halbleiterbauelements, wobei die elektrische Versorgungseinrichtung zumindest teilweise von dem thermoplastischen Kunststoff umhüllt wird. Durch diese Ausführung wird insbesondere die Herstellung der Beleuchtungsvorrichtung und deren Montage erleichtert. Bevorzugt werden der Träger und zumindest Teile der elektrischen Versorgungseinrichtung zuerst miteinander verbunden und anschließend mit dem thermoplastischen Kunststoff umhüllt. Auf dem Träger sind bevorzugt mehrere optische Halbleiterbauelemente oder LEDs angeordnet. Der Träger ist besonders bevorzugt ringförmig ausgebildet, so dass durch seine zentrale Bohrung ein Werkzeug durchführbar ist und er damit am Werkzeugende des Handgriffelements, rund um die Werkzeugaufnahme oder das Werkzeug anordenbar ist.

Der Aufbau der Beleuchtungsvorrichtung des Handgriffs gemäß einem Ausführungsbeispiel mit einem Grundkörper und zumindest einer im Grundkörper, zum Beispiel in dessen Wand, angeordneten Aufnahme und einem Lichtabgabefenster, das durch jenen Teil der Wand, der sich von der Aufnahme bis zum ersten Ende des Grundkörpers erstreckt, gebildet ist, hat den Vorteil, dass damit eine der Behandlungsstelle zugewandte Oberfläche geschaffen wird, die mechanischen Beschädigungen besser widerstehen kann als das weiche Kunstharz. Des Weiteren bietet die Oberfläche eine einfache Möglichkeit, das vom optischen Halbleiterbauelement abgestrahlte Licht aufzubereiten, in dem die Oberfläche des Grundkörpers als plane, polierte Oberfläche oder als optisches Element ausgebildet ist, zum Beispiel als konvexe oder konkave Linse. Ein bevorzugt einstückig mit dem Grundkörper ausgebildetes optisches Element hat den zusätzlichen Vorteil, dass es nicht an der Beleuchtungsvorrichtung befestigt werden muss und damit auch keine Gefahr besteht, dass es sich zum Beispiel aufgrund der bei der Sterilisation herrschenden Umgebungsbedingungen von der Beleuchtungsvorrichtung abhebt und in einem damit entstehenden Zwischenraum Keimen die Möglichkeit zur Ansiedlung bietet oder dass es sich vollkommen davon ablöst. An die Oberfläche kann auch in einfacher Weise ein Lichtleiter angeschlossen werden.

Die zumindest eine Aufnahme kann verschiedene Formen aufweise, sie kann zum Beispiel ein im wesentlichen zylindrischer Hohlraum sein, der nur unwesentlich größer als ein darin aufgenommenes optisches Halbleiterbauelement ist, wobei im Grundkörper oder in die Wand des Grundkörpers auch mehrere dieser Hohlräume gleichmäßig oder unregelmäßig verteilt sein können. Alternativ kann die Aufnahme größer, zum Beispiel kreisbogenförmig ausgebildet sein und sich um die Längsachse des Grundkörpers erstrecken, wobei wiederum auch mehrere, zum Beispiel zwei viertelkreisbogenförmige Aufnahmen vorhanden sein können. Schließlich kann die Aufnahme auch als ein durchgehender, rund um die Längsachse des Grundkörpers verlaufender Ring ausgebildet sein. Ist die Aufnahme derart ausgebildet, dass sie mehrere optische Halbleiterbauelemente aufnimmt, so ist damit eine besonders hohe Lichtintensität auf einer kleinen Fläche erzielbar.

Da der Grundkörper aus thermoplastischem Material hergestellt ist, ist er elektrisch nicht leitend. Des Weiteren muss das Lichtabgabefenster für die von dem zumindest einen optischen Halbleiterbauelement abgestrahlte Wellenlänge oder für ein Spektrum, das von einem in der Beleuchtungsvorrichtung angeordneten Farbkonversionsmaterial erzeugt wird, transparent sein.

Die mit dem Grundkörper verbindbare Abdeckvorrichtung kann in einer ersten Ausführungsform durch zumindest ein Vergussmaterial, insbesondere Kunstharze wie Epoxidharz, Silikonharze oder Polyamidharze gebildet sein. Diese Ausführungsform ist in ihrer Herstellung sehr einfach und kostengünstig. Sie ermöglicht auch mehrere, gegebenenfalls unterschiedliche optische Halbleiterbauelemente in jeweils einer eigenen Aufnahme anzuordnen und hermetisch zu verschließen, wobei besonders bevorzugt die Anordnung der optischen Halbleiterbauelemente unregelmäßig ist, so dass spezielle Lichtverteilungsmuster realisierbar sind. Bei dieser Ausführungsform kann zumindest ein Teil der Vergussmasse in der Aufnahme aufgenommen sein.

In einer zweiten Ausführungsform umfasst die Abdeckvorrichtung zumindest einen Verschlusskörper, der bevorzugt zylindrisch, ring-, rohr-, stab- oder plattenförmig geformt ist. Die Abdeckvorrichtung besteht zum Beispiel aus Kunststoff, insbesondere aus Polyphenylsulfon, Polyethersulfon, Polycarbonat oder Polyamid, Glas oder Keramik. Auch der Träger kann als Abdeckvorrichtung für die LEDs dienen. Die Verbindung des Grundkörpers mit der Abdeckvorrichtung kann durch Verschweißen, zum Beispiel Ultraschall- oder Laserverschweißen, Verpressen, Verkleben oder mittels Dichtelementen, zum Beispiel O-Ringen oder Gummiringen erfolgen. Bevorzugt wird das Laser-Durchstrahl-Schweißverfahren verwendet, bei dem eines der beiden zu verbindenden Bauteile für die Laserstrahlung transparent ist und das andere Bauteil die Laserstrahlung absorbiert. Durch die absorbierte Strahlung schmilzt dieses Bauteil oberflächlich auf und überträgt einen Teil der Wärme auf das transparente Bauteil, das ebenfalls oberflächlich aufschmilzt, so dass sich im Bereich der einander kontaktierenden Oberflächen die beiden Bauteile beim anschließenden Abkühlen verbinden. Vorteilhafterweise sind für die Anwendung dieses Verfahrens beide Bauteile aus dem gleichen Material hergestellt.

Bevorzugt ist die Abdeckvorrichtung jedoch wie der Grundkörper auch aus einem thermoplastischen Kunststoff hergestellt, zum Beispiel aus statistische-Propylen-Copolymere (PPR), Polycarbonate (PC), Polymethylenpenten (PMP), Cyclooleofin-Copolymere (COC) oder Polyphenylsulfon (PPSU). Sind der Grundkörper und die Abdeckvorrichtung zweiteilig ausgebildet, so können sie durch Verschweißen, Verkleben, Verpressen, Verschmelzen oder mittels Dichtelementen miteinander verbunden werden. Besonders bevorzugt sind die Abdeckvorrichtung und der Grundkörper als einteilige Umhüllung ausgebildet, so dass keine zusätzliche Verbindung zwischen den beiden Bauteilen notwendig ist, wodurch eine besonders hohe Dichtwirkung für das zumindest eine optische Halbleiterbauelement erreicht wird. Der Grundkörper und die Abdeckvorrichtung sind bei dieser einteiligen Ausführungsform bevorzugt durch Spritzgießen hergestellt.

Wie bereits angeführt, ist es nicht zwingend notwendig, dass der Verschlusskörper der Abdeckvorrichtung und der Grundkörper aus denselben Materialien bestehen. Werden unterschiedliche Materialien verwendet, so sollen diese jedoch einen ähnlichen oder annähernd gleichen Wärmeausdehnungskoeffizienten haben, so dass insbesondere bei Erwärmung der Beleuchtungsvorrichtung während der Sterilisation keine Risse oder undichte Stellen zwischen den beiden Bauteilen entstehen, durch die Wasserdampf, Chemikalien oder andere Verschmutzungen eindringen können.

Das optische Halbleiterbauelement ist bevorzugt als Halbleiter-Chip ausgebildet, der insbesondere Teil eines Beleuchtungselements ist, zum Beispiel einer Leuchtdiode (LED). Das zumindest eine optische Halbleiterbauelement oder die LED sind auf einem Träger, zum Beispiel einer Platine oder einer Multilayer-Keramik, angeordnet. Bevorzugt sind sie jedoch ohne Träger direkt in die Sacklochbohrung oder in die Aufnahme eingesetzt und darin gekapselt oder darin vergossen oder durch ein Abdeckteil hermetisch verschlossen. Diese Ausführungsform hat den Vorteil einer besonders geringen Bauhöhe und damit eines besonders geringen Platzbedarfs im oder am Handgriff.

Die elektrische Versorgungseinrichtung umfasst in einem ersten Ausführungsbeispiel zumindest einen ersten und zweiten elektrischen Kontakt zur Stromversorgung des zumindest einen optischen Halbleiterbauelements. Die Kontakte können zum Beispiel aus Kontaktstiften oder aus Litzen gebildet sein. Sind mehrere Halbleiterbauelemente vorhanden, so können diese in Serie oder parallel angeordnet sein, so dass entsprechend mehrere elektrische Kontakte vorhanden sind. Die Verbindung zwischen dem zumindest einen optischen Halbleiterbauelement und den elektrischen Kontakten kann durch Bondingdrähte oder über Platinenleitungen erfolgen.

Ist die Abdeckvorrichtung durch zumindest ein Vergussmaterial gebildet, so werden bevorzugt die elektrischen Kontakte zuerst mit dem zumindest einen optischen Halbleiterbauelement verbunden, in die Aufnahme zumindest teilweise eingebracht und anschließend vergossen. Alternativ ist es auch möglich, die Kontakte nicht durch das Vergussmaterial zu führen, sondern durch den Grundkörper, wobei die Abdichtung in gleicher Weise erfolgt wie dies im Folgenden für einen Verschlusskörper einer Abdeckvorrichtung beschrieben.

Umfasst die Umhüllung bzw. die Abdeckvorrichtung zumindest einen Grundkörper und einen Verschlusskörper, so sind die zumindest zwei elektrischen Kontakte durch Bohrungen entweder im Verschluss- oder im Grundkörper geführt, wobei die Bohrungen bis in die Aufnahme / den hermetisch abgedichteten Innenraum reichen. Die Abdichtung der elektrischen Kontakte kann wiederum durch Verschweißen, zum Beispiel Ultraschall- oder Laserverschweißen, Verpressen, Verkleben oder mittels Dichtelementen erfolgen.

Ist die Umhüllung, d.h. die Abdeckvorrichtung und der Grundkörper, einteilig ausgebildet, so wird das zumindest eine optische Halbleiterbauelement vor dem Umhüllen mit den elektrischen Kontakten verbunden und anschließend das optische Halbleiterbauelement mit dem thermoplastischen Kunststoff umgeben. Bevorzugt werden der Verschluss- oder Grundkörper durch Spritzgießen hergestellt, wobei die elektrischen Kontakte vor Beginn der Spritzgießherstellung in die Spritzgießform eingebracht und mitverspritzt werden, so dass sie in Bohrungen oder Kanälen im thermoplastischen Kunststoff aufgenommen sind, die exakt dem Durchmesser der Kontakte entsprechen, wodurch eine äußerst zuverlässige Abdichtung erreicht wird.

In einem zweiten Ausführungsbeispiel ist eine induktive elektrische Versorgungseinrichtung zur Stromversorgung des zumindest einen optischen Halbleiterbauelements vorgesehen, mit zumindest einer ersten Spule, die im hermetisch abgedichteten Innenraum der Beleuchtungsvorrichtung angeordnet ist, und zumindest einer zweiten Spule, die außerhalb des hermetisch abgedichteten Innenraums angeordnet ist. Bevorzugt sind zwischen der ersten Spule und dem zumindest einen optischen Halbleiterbauelement weitere elektronische Bauelemente, wie zum Beispiel Gleichrichter, Stromregler, AC-DC-Wandler, Kondensatoren oder Widerstände vorgesehen, um das zumindest eine optische Halbleiterbauelement mit der benötigten Gleichspannung zu versorgen bzw. eine konstante Stromversorgung sicherzustellen. Ist aufgrund der beengten Platzverhältnisse eine Verwendung dieser elektronischen Bauteile nicht möglich, so kann die induktive Versorgungseinrichtung oberhalb der so genannten Flimmerfrequenz (entsprechend etwa 25 Hz Wechselspannung) betrieben werden, wodurch für das menschliche Auge der Eindruck einer konstanten, nicht unterbrochenen Lichtabgabe des zumindest einen optischen Halbleiterbauelements entsteht.

Als primäre Stromquelle, mit der die elektrischen Kontakte oder die induktive Versorgungseinrichtung verbunden oder verbindbar sind, kann eine Batterie, ein Akkumulator oder das Stromnetz dienen. Bevorzugt wird die von den genannten Stromquellen zur Verfügung gestellte Energie entsprechend den Anforderungen der Halbleiterbauelemente mittels elektronischer oder elektrischer Bauelemente aufbereitet.

In einem bevorzugten Ausführungsbeispiel ist die Beleuchtungsvorrichtung als Ringlicht ausgebildet, das bevorzugt sehr nahe am oder direkt um den Werkzeughalter oder um das Werkzeug angebracht ist. Damit ist kein Lichtleiter notwendig, der das Licht durch den Handgriff an das werkzeugseitige Ende des Handgriffs leitet, wodurch die Strahlungsverluste deutlich reduziert werden. Bei der als Ringlicht ausgebildeten Beleuchtungsvorrichtung haben zumindest der Grundkörper, gegebenenfalls auch der Träger und die Abdeckvorrichtung eine Bohrung, in der das Werkzeug oder die Werkzeugaufnahme aufgenommen oder durchgeführt werden können.

In einem anderen Ausführungsbeispiel ist die Umhüllung der Beleuchtungsvorrichtung aus sterilisierbarem thermoplastischen Kunststoff hergestellt, insbesondere aus thermoplastischen Kunststoff mit einem Wiedererweichungspunkt über 120°C. Unter dem Begriff "wiederholtes Sterilisieren widerstehendes Material", "sterilisierbares Material" oder "sterilisierbarer Kunststoff wird jedes Material verstanden, das auch nach mehrmaligem Einbringen in eine aggressive Umgebung mit einer Temperatur von zumindest 120°C, bevorzugt von 130°C, besonders bevorzugt von 134°C und mit Wasserdampf und / oder in eine Umgebung mit Chemikalien, insbesondere Desinfektionsmittel keine wesentlichen Veränderungen oder Schäden aufweist, so dass ein durch ein derartiges Material umschlossener und von der Umwelt hermetisch abgeschlossener Innenraum auch nach mehrmaligem Einbringen des Materials in diese aggressive Umgebung hermetisch verschlossen bleibt. Im Innenraum aufgenommene Bauteile, insbesondere optische Halbleiterbauelemente sind somit durch eine Kapselung mit einem derartigen Material sowohl während des Sterilisationsvorganges als auch danach vor Wasserdampf, Chemikalien, Partikeln, Schmiermitteln oder anderen Verschmutzungen geschützt und können so ihre Funktionsfähigkeit aufrechterhalten. Insbesondere wird unter dem Begriff "sterilisierbares Material" oder "sterilisierbarer Kunststoff" jedes Material oder jeder Kunststoff verstanden, dessen Schmelzpunkt oder Wiedererweichungspunkt über 120°C liegt.

Die Erfindung wird nachfolgend anhand von bevorzugten Ausführungsbeispielen und Bezug nehmend auf die beigefügten Zeichnungen erläutert:
Figur 1 zeigt als Explosionsdarstellung ein erstes Ausführungsbeispiel der Beleuchtungsvorrichtung eines erfindungsgemäßen medizinischen Handgriffs.
Figur 2 zeigt einen Längsschnitt durch die zusammengesetzte Beleuchtungsvorrichtung der Figur 2.
Figur 3 zeigt ein zweites Ausführungsbeispiel der Beleuchtungsvorrichtung eines erfindungsgemäßen medizinischen Handgriffs.
Figur 4 zeigt einen erfindungsgemäßen medizinischen Handgriff in Form eines dentalen Zahnsteinentfernungsgeräts.
Figur 5 zeigt ein drittes Ausführungsbeispiel der Beleuchtungsvorrichtung eines erfindungsgemäßen medizinischen Handgriffs.

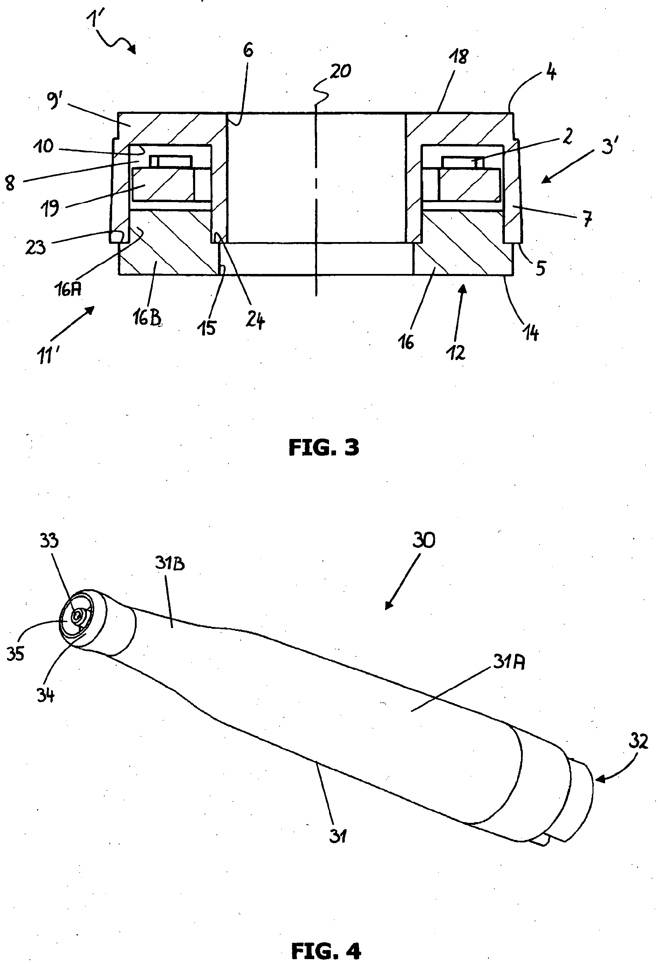

In Figur 4 ist beispielhaft für einen medizinischen Handgriff 30 ein Zahnsteinentfemungsgerät, oftmals auch als Scaler bezeichnet, dargestellt. Selbstverständlich ist der Begriff Handgriff jedoch nicht auf Scaler beschränkt sondern schließt auch alle anderen medizinisch, chirurgisch, orthopädisch, dental oder kosmetisch verwendbaren Handgriffe mit ein, insbesondere auch Handgriffe die gebogen sind oder gewinkelt zueinander angeordnete Abschnitte aufweisen, Handgriffe mit unterschiedlichen Antrieben oder Antriebsenergien, zum Beispiel mit Druckluft, Wasser oder Strom betriebene Handgriffe, Handgriffe mit unterschiedlichen Werkzeugen wie Bohrer, Sägen, Fräsen, Bürsten, Prophy-Cups, Reamer, Shaver etc., medizinische Handgriffe zur Abgabe von Laserstrahlung oder Handgriffe, deren primäre Aufgabe die Abgabe von Licht zur Beleuchtung oder zur diagnostischen, therapeutischen oder prophylaktischen Behandlung ist. Unter dem Begriff Handgriff sind des Weiteren auch Teile solcher Handgriffe und Bauelemente wie Motore, Kupplungen, Adapter oder Zwischenstücke zur Verbindung von Bauteilen eines medizinischen Geräts zu verstehen.

Der Scaler umfasst eine längliche, zylindrische, innen hohle Außenhülse 31, die in einen Griffteil 31A und einen Kopfteil 31B unterteilt ist. Im Inneren der Außenhülse 31 können verschiedene Funktionsteil angeordnet sein, so zum Beispiel eine Antriebseinheit, Leitungen für Antriebs- und / oder Kühlmedien, Vibrationen dämpfende Einbauten, Regel- und Stelleinrichtungen usw. An dem dem Kopfteil 31 B gegenüberliegenden Ende des Griffteils 31 A ist eine Anschlussvorrichtung 32, zum Anschluss des Handgriffs 30 an eine Energiequelle, zum Beispiel an eine Druckluft- oder Stromquelle, an eine oder mehrere Kühlmedienquellen und gegebenenfalls an eine Steuervorrichtung vorgesehen. Da die Anschlussvorrichtung 32 bzw. die verschiedenen Arten von Anschlussvorrichtungen, die mit einem derartigen Handgriff 30 verwendbar sind, bekannt sind, wird darauf nicht weiter eingegangen.

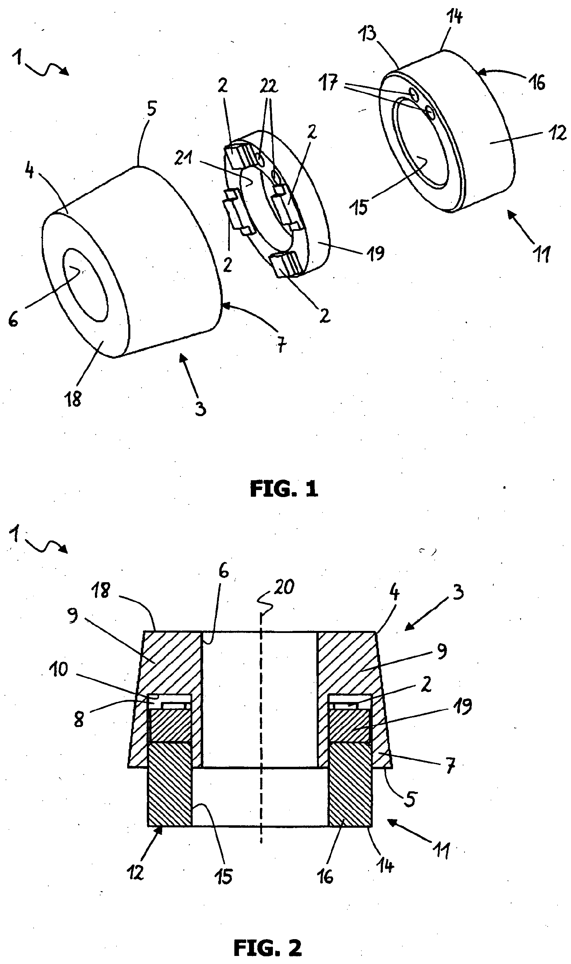

Am distalen Ende des Kopfteils 31 B ist eine Werkzeugaufnahme 33 in Form eines hohlen Schaftes mit Innengewinde erkennbar, in die das Behandlungswerkzeug einschraubbar ist. Die Werkzeugaufnahme 33 ist vom hülsenförmig ausgebildeten, zylindrischen Vorderende 34 des Kopfteils 31B umgeben, wobei das zylindrische Vorderende 34 von der Werkzeugaufnahme 33 beabstandet ist, wodurch ein Hohlraum 35 entsteht. In diesen Hohlraum 35 ist eine als Ringlicht ausgebildete Beleuchtungsvorrichtung 1, wie sie zum Beispiel in den Figuren 1 - 3 dargestellt ist, einsetzbar. Die Beleuchtungsvorrichtung 1 ist somit im Wesentlichen im Handgriff 30 aufgenommen, wobei die Lichtabgabefläche 18 in etwa plan mit dem Vorderende 34 abschließt. Die Werkzeugaufnahme 33 ragt in oder durch eine oder mehrere Bohrungen 6, 15 der Beleuchtungsvorrichtung 1, so dass sie für den Anwender frei zugänglich ist und dieser das Behandlungswerkzeug problemlos in die Werkzeugaufnahme 33 einsetzen oder daraus lösen kann.

Die Beleuchtungsvorrichtung 1 ist gemäß den Figuren 1 - 3 dreiteilig aufgebaut und besteht aus einem Grundkörper 3, einer Abdeckvorrichtung 11 und einem Träger 19 in Form einer Platine. Der Grundkörper 3 hat eine schwach kegelförmige Außenform mit einem ersten Ende 4, das einen geringeren Durchmesser hat als das zweite Ende 5. Zwischen den beiden Enden 4, 5 verläuft eine konzentrisch zur Längsachse 20 der Beleuchtungsvorrichtung 1 angeordnete Bohrung 6, in die wie oben beschrieben die Werkzeugaufnahme 33 ragt, wenn die Beleuchtungsvorrichtung 1 am Handgriff 30 befestigt ist. Rund um die Längsachse 20 und rund um die Bohrung 6 verläuft eine Wand 7, in der eine ringförmige Aufnahme oder Sacklochbohrung 8 vorgesehen ist.

Jener Teil der Wand 7, der sich vom Boden 10 der Aufnahme oder Sacklochbohrung 8 bis zum ersten Ende 4 des Grundkörpers 3 erstreckt, bildet ein Lichtabgabefenster 9. Das Lichtabgabefenster 9 ist somit ein einstückig ausgeführter, integraler Teil des Grundkörpers 3, der aus einem Material besteht, das transparent für das von den LEDs 2 abgestrahlte oder konvertierte Licht ist. Das Lichtabgabefenster 9 endet am ersten Ende 4 in einer Lichtabgabefläche 18, die in den Figuren 1 - 3 als plane Fläche ausgebildet ist, auf der gegebenenfalls ein optisches Element, zum Beispiel eine Sammellinse anbringbar ist. Alternativ kann die Lichtabgabefläche 18 selbst als optisches Element dienen, d.h. zum Beispiel als Sammellinse geformt sein. Der Grundkörper 3 besteht aus Kunststoff, insbesondere aus transparentem, thermoplastischem Kunststoff, Polyphenylsulfon, Polyethersulfon, Polycarbonat oder Polyamid, oder aus Glas. Der Grundkörper 3 der Figur 1 hat in etwa eine Länge von 5 - 10 mm, einen Außendurchmesser am ersten Ende 4 von in etwa 11 mm und einen Innendurchmesser der Bohrung 6 am ersten Ende 4 von in etwa 5 mm.

Auf dem ringförmigen Träger 19 sind vier optische Halbleiterbauelemente 2 in Form von Leuchtdioden (LEDs) in einem regelmäßigen Abstand von etwa 90° befestigt. Es können dabei identische LEDs verwendet werden oder LEDs, die unterschiedliche Wellenlängen abstrahlen, so dass insgesamt ein im Wesentlichen weißes Licht emittiert wird. Der Träger 19 wird von einer ersten zentrischen Bohrung 21 und zwei weiteren, kleineren, exzentrischen Bohrungen 22 durchsetzt. Wie insbesondere aus den Figuren 2 und 3 ersichtlich ist, ist der Außendurchmesser des Trägers 19, der Durchmesser der Bohrung 21 und somit die Wandstärke der ringförmigen Trägerwand derart bemessen, dass der Träger 19 mit den LEDs 2 in die Sacklochbohrung 8 einsetzbar ist. Durch die Bohrungen 22 verläuft jeweils ein elektrischer Kontakt als Teil der elektrischen Versorgungseinrichtung zur Stromversorgung der LEDs 2, die in Serie geschalten sind.

Die Abdeckvorrichtung 11 ist als ringförmiger Verschlusskörper 12 mit einer zentralen Bohrung 15, die vom zweiten Ende 14 zum ersten Ende 13 reicht, ausgebildet. Durch Bohrung 15 ragt, genauso wie durch Bohrung 21 des Trägers 19 die Werkzeugaufnahme 33, wenn die Beleuchtungsvorrichtung 1 am Handgriff 30 befestigt ist. In die ringförmigen Wand 16 der Abdeckvorrichtung 11 sind zwei kleine Bohrungen 17 zur Aufnahme der elektrischen Kontakte der elektrischen Versorgungseinrichtung eingebracht. In montiertem Zustand schließen die Bohrungen 17 an die Bohrungen 22 des Trägers 19 an. Die Abdeckvorrichtung 11 ist aus Kunststoff, insbesondere aus transparentem, thermoplastischem Kunststoff, Polyphenylsulfon, Polyethersulfon, Polycarbonat oder Polyamid, Glas oder Keramik hergestellt. Die Abdeckvorrichtung 11 der Figur 2 hat in etwa eine Länge von 1 - 5 mm.

Wie aus den Figuren 2 und 3 erkennbar ist, ist ein Teil der Abdeckvorrichtung 11 in der Sacklochbohrung 8 des Grundkörpers 3 aufgenommen. Um das Einbringen der Abdeckvorrichtung 11 in die Sacklochbohrung 8 zu erleichtern, weist das erste Ende 13 an seinem Außenumfang und am Umfang der Bohrung 15 Fasen auf. Durch eine dauerhaft dichte Verbindung der Abdeckvorrichtung 11 mit dem Grundkörper 3 entsteht aus der Sacklochbohrung 8 ein hermetisch abgedichteter Innenraum, in dem die LEDs 2 angeordnet sind. Die Verbindung kann durch Verpressen, Verkleben oder mittels Dichtelementen, zum Beispiel O-Ringen oder Gummiringen erfolgen. Bevorzugt erfolgt die Verbindung durch Verschweißen, besonders bevorzugt durch Laserverschweißen. Bei der Abdichtung mittels Dichtelementen können zur Aufnahme der Dichtelemente Ringnuten in der Wand 16 der Abdeckvorrichtung 11 und / oder in den Wänden der Sacklochbohrung 8 vorgesehen sein.

Bei Betrieb emittieren die LEDs 2 Strahlung in den hermetisch abgedichteten Innenraum, wobei die Strahlung über das transparente Lichtabgabefenster 9 und die Lichtabgabeoberfläche 18 an die Umgebung abgestrahlt wird. Im hermetisch abgedichteten Innenraum können gegebenenfalls weitere Funktionselemente zur Verbesserung der Lichtabgabe enthalten sein, so zum Beispiel Lumineszenzfarbstoffe enthaltende Konvertierungspasten zur Umwandlung der von den LEDs 2 abgestrahlten Wellenlänge, Filter oder Reflektoren zur Lenkung der Strahlung in Richtung des Lichtabgabefensters 9. Auch diese Funktionselemente sind somit vor Feuchtigkeit, Chemikalien und anderen schädlichen Umgebungseinflüssen geschützt.

Die Beleuchtungsvorrichtung 1' der Figur 3 unterscheidet sich von der Beleuchtungsvorrichtung 1 durch ihre deutlich geringere Bauhöhe, die durch geringer Längenabmessungen - bezogen auf die Längsachse 20 - des Grundkörpers 3' und der Abdeckvorrichtung 11' erzielt wird. Entsprechend ist auch das Lichtabgabefenster 9' schmäler ausgebildet. Um dennoch eine zuverlässige Kapselung des die Sacklochbohrung 8 umfassenden, hermetisch abgedichteten Innenraums zu erhalten, besteht die Wand 16 der Abdeckvorrichtung 11' aus einem ersten Abschnitt 16A mit einem geringeren Durchmesser und einem zweiten, flanschartigen Abschnitt 16B mit einem größeren Durchmesser. Die durch den flanschartigen Abschnitt 16B gebildeten Ringschultern 23 und 24 dienen als Auflager für Grundkörper 3' und insbesondere als eine hervorragende Verbindungsstelle zum Verschweißen des Grundkörpers 3' mit der Abdeckvorrichtung 11'.

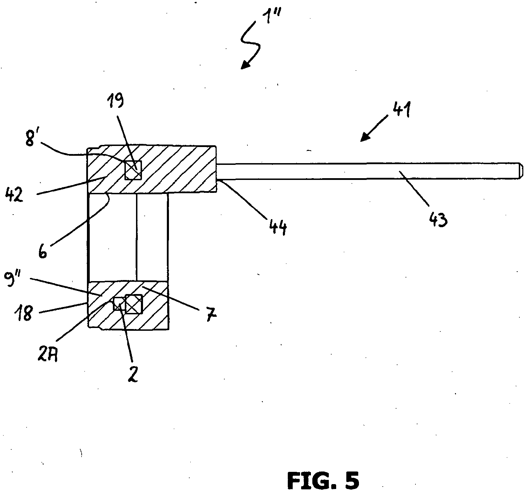

Die Beleuchtungsvorrichtung 1" gemäß Figur 5 umfasst wiederum ein ringförmiges, bevorzugt als Platine oder als Multilayer-Keramik ausgebildetes, Trägerelement 19 mit einer zentralen Bohrung, auf dem mehrere optische Halbleiterbauelemente 2 angebracht sind. Eine elektrische Versorgungseinrichtung 41, die zwei metallischen Kontaktstifte 43 (nur einer ist dargestellt) umfasst, verbindet die optischen Halbleiterbauelemente 2 mit einer Stromquelle. Der Träger 19, die optischen Halbleiterbauelemente 2 sowie ein Teil der elektrischen Kontakte 43 sind von einer Umhüllung 42 aus transparentem, thermoplastischem Kunststoff umgeben. Rund um die elektrischen Kontakte 43 weist die Umhüllung 42 einen Fortsatz auf, der von der ansonsten hohlzylindrisch geformten Beleuchtungsvorrichtung 1" absteht.

Bei dem für die Umhüllung 42 verwendeten transparenten, thermoplastischen Kunststoff handelt es sich bevorzugt um ein statistisches Propylen-Copolymer (PPR), insbesondere um eine nukleierte statistische Polypropylen-Verbindung (nucleated random copolymer polypropylen), die zum Beispiel von der Firma Total Petrochemicals unter der Bezeichnung PPR 9220 vertrieben wird. Selbstverständlich kann die Umhüllung 42 jedoch auch aus anderen thermoplastischen Kunststoffen gebildet sein.

Wie aus der Figur 5 zu erkennen ist, ist die Umhüllung 42 der optischen Halbleiterbauelemente 2 einteilig ausgeführt, so dass es keine Trennung der Umhüllung in einen Grundkörper und eine Abdeckvorrichtung gibt. Damit entfällt auch die Notwendigkeit, den Grundkörper und die Abdeckvorrichtung miteinander zu verbinden, womit die Gefahr von Undichtheiten an der Verbindungsstelle eliminiert ist. Selbstverständlich ist es jedoch auch möglich, den Grundkörper und die Abdeckvorrichtung als separate Bauteile aus thermoplastischem Kunststoff zu fertigen und diese anschließend miteinander zu verbinden. Der Aufbau einer derartigen zwei- oder mehrteiligen Beleuchtungsvorrichtung ähnelt oder gleicht dann zum Beispiel den Beleuchtungsvorrichtungen 1 und 1' der Figuren 1 - 3.

Die Umhüllung der Bauteile mit dem thermoplastischen Kunststoff erfolgt durch Verflüssigen des in fester Form vorliegenden thermoplastischen Kunststoffs, insbesondere durch Erwärmen, Umgießen der Bauteile mit dem flüssigen Kunststoff und Aushärten des thermoplastischen Kunststoffs mit den darin eingebetteten Bauteilen. Das Umgießen erfolgt bevorzugt unter Druck, besonders bevorzugt mittels Spritzgießverfahrens.

Der Träger 19 und die optischen Halbleiterbauelemente 2 sind in einer Aufnahme 8' der Umhüllung 42 angeordnet. Die elektrischen Kontakte 43 sind in Bohrungen 44, die von einem Ende der Umhüllung 42 bis zu den optischen Halbleiterbauelementen 2 verlaufen, aufgenommen. Der Träger 19, die optischen Halbleiterbauelemente 2 und die elektrischen Kontakte 43 sind von dem thermoplastischen Kunststoff derart dicht umhüllt, dass Flüssigkeiten oder Gase nicht bis zu den optischen Halbleiterbauelemente 2 und dem Träger vordringen können. Die Aufnahme 8' ist somit als hermetisch abgedichteter Innenraum ausgebildet.

Aus der Figur 5 ist des Weiteren zu erkennen, dass der thermoplastische Kunststoff der Umhüllung 42 die Strahlung emittierenden Flächen 2A des optischen Haibteiterbaueigments 2 direkt kontaktiert. An diese Flächen 2A bzw. an die Aufnahme 8' schließt das Lichtabgabefenster 9' an, das in einer planen oder gebogenen Lichtabgabefläche 18 endet.

Entspredhend den Beleuchtungsvorrichtungen 1 und 1' weist auch die Beleuchtungsvorrichtung 1' eine Bohrung 6 auf, in die die Werkzeugaufnahme 33 und ein Werkzeug ragen, wenn die Beleuchtungsvorrichtung 1' am Handgriff 30 befestigt ist. Rund um die Bohrung 6 verläuft eine ringförmige, die Umhüllung 42 bildenden Wand 7.

Die Erfindung ist nicht auf die beschriebenen Ausführungsbeispiele beschränkt, sondern umfaßt alle Ausführungsmöglichkelten, die das prinzipielle, sinngemäße Funktionsprinzip der Erfindung nicht verändern. Insbesondere ist die Form der gesamten Beteucntungsvomchtung oder von Teilen davon variabel und nicht auf die dargestellte ringförmige, zylindrische oder kegelförmige Form beschränkt. Abhängig vom vorhandenen Platz, einem gewünschten Lichtverteilungsmuster oder anderen Anforderungen sind zum Beispiel auch rechteckige oder polygonale Formen möglich. Es wird auch darauf verwiesen, dass die Beteuchtungsvorrichtung nicht wie den Figuren 1-3 und 5 dargestellt zwingend als Ringlicht, das ein Behandlungswerkzeug, eine Werkzeugaufnahme oder ein anderes Bauteil des Handgriffs Umgibt und mit den Bohrungen 6, 15, 21 versehen ist, ausgebildet sein muss. Selbstverständlich kann eine Beleuchtungsvorrichtung gemäß der Erfindung auch ohne diese Bohrungen 6, 15, 21 hergestellt werden und beispielsweise an die Lichtabgabefläche 18 ein Lichtleiter angeschlossen werden, der das Licht an das werkzeugseitige Ende oder an eine andere gewünschte Lichtabgabestelle des Handgriffs weiterleitet.

## Patentansprüche

1. Verfahren zur Herstellung eines medizinischen Handgriffs (30) mit einer Beleuchtungsvorrichtung (1, 1', 1 "), welche zumindest ein als Strahlungsquelle ausgebildetes optisches Halbleiterbauelement (2), eine als Träger (19) für das zumindest eine optische Halbleiterbauelement (2) ausgebildete Platine oder Multilayer-Keramik und eine durch elektrische Kontakte (43) gebildete elektrische Versorgungseinrichtung (41) zur Stromversorgung des zumindest einen optischen Halbleiterbauelements (2) umfasst, wobei das Verfahren durch folgende Schritte gekennzeichnet ist:
• Zur Verfügung stellen eines medizinischen Handgriffs (30),
• Zur Verfügung stellen der Beleuchtungsvorrichtung (1, 1', 1"),
• Umhüllen der Beleuchtungsvorrichtung (1, 1', 1") mit einem transparenten, thermoplastischen Kunststoff, derart, dass eine Umhüllung (42) entsteht, welche das zumindest eine optische Halbleiterbauelement (2) und die Platine oder Multilayer-Keramik allseitig und die elektrischen Kontakte (43) teilweise umgibt, und
• Befestigen des zumindest einen, umhüllten optischen Halbleiterbauelements (2) in oder an dem medizinischen Handgriff (30).

2. Verfahren zur Herstellung eines medizinischen Handgriffs (30) nach Anspruch 1, **dadurch gekennzeichnet, dass**
das Umhüllen durch zur Verfügung stellen eines festen thermoplastischen Kunststoffs, Verflüssigen des thermoplastischen Kunststoffs und Aushärten des thermoplastischen Kunststoffs erfolgt.

3. Verfahren zur Herstellung eines medizinischen Handgriffs (30) nach Anspruch 1 oder 2, **dadurch gekennzeichnet, dass**
das Umhüllen durch Erhitzen des thermoplastischen Kunststoffs erfolgt, bevorzugt auf Temperaturen von maximal etwa 310°C, besonders bevorzugt auf Temperaturen von maximal etwa 280°C.

4. Verfahren zur Herstellung eines medizinischen Handgriffs (30) nach einem der vorhergehenden Ansprüche, **dadurch gekennzeichnet, dass**
vor dem Umhüllen mit dem thermoplastischen Kunststoff das zumindest eine optische Halbleiterbauelement (2) in einer Form angeordnet wird und der thermoplastische Kunststoff in die Form eingebracht wird.

5. Verfahren zur Herstellung eines medizinischen Handgriffs (30) nach Anspruch 4, **dadurch gekennzeichnet, dass**
der thermoplastische Kunststoff unter Druck in die Form eingespritzt wird.

6. Verfahren zur Herstellung eines medizinischen Handgriffs (30) nach einem der vorhergehenden Ansprüche, **dadurch gekennzeichnet, dass**
das zumindest eine optische Halbleiterbauelement (2) derart von dem thermoplastischen Kunststoff umhüllt wird, dass der thermoplastische Kunststoff entweder zumindest die Strahlung emittierende Fläche (2A) des zumindest einen optischen Halbleiterbauelements (2) oder an die Strahlung emittierende Fläche (2A) anschließende Bauteile kontaktiert.

7. Verfahren zur Herstellung eines medizinischen Handgriffs (30) nach einem der vorhergehenden Ansprüche, **dadurch gekennzeichnet, dass**
die Umhüllung (42) einteilig hergestellt wird.

8. Medizinischer Handgriff (30) mit einer Beleuchtungsvorrichtung (1, 1', 1"), welche zumindest ein als Strahlungsquelle ausgebildetes optisches Halbleiterbauelement (2), eine als Träger (19) für das zumindest eine optische Halbleiterbauelement (2) ausgebildete Platine oder Multilayer-Keramik und eine durch elektrische Kontakte (43) gebildete elektrische Versorgungseinrichtung (41) zur Stromversorgung des zumindest einen optischen Halbleiterbauelements (2) umfasst, **dadurch gekennzeichnet dass**
die Beleuchtungsvorrichtung (1, 1', 1") eine Umhüllung (42) mit einem transparenten, thermoplastischen Kunststoff aufweist, welche das zumindest eine optische Halbleiterbauelement (2) und die Platine oder Multilayer-Keramik allseitig und die elektrischen Kontakte (43) teilweise umgibt.

9. Medizinischer Handgriff (30) nach Anspruch 8, **dadurch gekennzeichnet, dass** das zumindest eine optische Halbleiterbauelement (2) derart von dem thermoplastischen Kunststoff umhüllt ist, dass der thermoplastische Kunststoff entweder zumindest die Strahlung emittierende Fläche (2A) des zumindest einen optischen Halbleiterbauelements (2) oder an die Strahlung emittierende Fläche (2A) anschließende Bauteile kontaktiert.

10. Medizinischer Handgriff (30) nach Anspruch 8 oder 9, **dadurch gekennzeichnet, dass** die durch den thermoplastischen Kunststoff gebildete Umhüllung (42) einteilig ausgebildet ist.

11. Medizinischer Handgriff (30) nach Anspruch 8 oder 9, **dadurch gekennzeichnet, dass** die durch den thermoplastischen Kunststoff gebildete Umhüllung zwei- oder mehrteilig ausgebildet ist.

12. Medizinischer Handgriff (30) nach Anspruch 11, **dadurch gekennzeichnet, dass** die Umhüllung aus dem transparenten, thermoplastischen Kunststoff umfasst:
• einen Grundkörper (3, 3') mit einem ersten Ende (4), einem zweiten Ende (5) und zumindest einer Aufnahme (8, 8'), wobei die zumindest eine Aufnahme (8, 8') sich vom zweiten Ende (5) des Grundkörpers (3, 3') in Richtung des ersten Endes (4) erstreckt,
• ein Lichtabgabefenster (9, 9', 9"), das durch jenen Teil des Grundkörpers (3, 3'), der sich von der zumindest einen Aufnahme (8, 8') bis zum ersten Ende (4) des Grundkörpers (3, 3') erstreckt, gebildet ist,
• eine Abdeckvorrichtung (11, 11'), die derart mit dem Grundkörper (3, 3') verbunden ist, dass die zumindest eine Aufnahme (8, 8') einen hermetisch abgedichteten Innenraum bildet,
wobei das zumindest eine optische Halbleiterbauelement (2) in dem hermetisch abgedichteten Innenraum angeordnet ist.

13. Medizinischer Handgriff (30) nach Anspruch 12, **dadurch gekennzeichnet, dass** zumindest ein Teil der Abdeckvorrichtung (11, 11') in der zumindest einen Aufnahme des Grundkörpers (3, 3') aufgenommen ist.

14. Medizinischer Handgriff (30) nach Anspruch 12 oder 13, **dadurch gekennzeichnet, dass**
die Abdeckvorrichtung (11 11') einen Verschlusskörper (12) mit einem ersten Ende (13) und einem zweiten Ende (14) umfasst, wobei der Verschlusskörper (12) bevorzugt mit einer vom ersten Ende (13) zum zweiten Ende (14) reichenden Bohrung (15) und einer die Bohrung (15) umschließenden Wand (16) ausgebildet ist.

15. Medizinischer Handgriff (30) nach einem der Ansprüche 8 - 14, **dadurch gekennzeichnet, dass**
die Umhüllung (42) der Beleuchtungsvorrichtung (1, 1') aus thermoplastischem Kunststoff hergestellt ist, dessen Wiedererweichungspunkt über 120°C liegt.

16. Medizinischer Handgriff (30) nach einem der Ansprüche 8 - 15, **dadurch gekennzeichnet, dass**
die elektrischen Kontakte (43) der elektrischen Versorgungseinrichtung (41) durch Bohrungen (17, 22, 44) in der Umhüllung (3, 3', 11, 11', 42) mit dem zumindest einen optischen Halbleiterbauelement (2) verbunden sind.

17. Medizinischer Handgriff (30) nach einem der Ansprüche 8 - 15, **gekennzeichnet durch** eine induktive elektrische Versorgungseinrichtung mit zumindest einer ersten Spule, die im hermetisch abgedichteten Innenraum angeordnet ist, und mit zumindest einer zweiten Spule, die außerhalb des hermetisch abgedichteten Innenraums vorgesehen ist, wobei bevorzugt zwischen der ersten Spule und dem zumindest einen optischen Halbleiterbauelement weitere elektronische Bauelemente, insbesondere
Gleichrichter, Stromregler, AC-DC-Wandler, Kondensatoren oder Widerstände vorgesehen sind.

18. Medizinischer Handgriff (30) nach einem der Ansprüche 8 - 17, **dadurch gekennzeichnet, dass**
die Platine oder Multilayer-Keramik ringförmig mit einer zentralen Bohrung ausgebildet ist, auf der mehrere optische Halbleiterbauelemente (2) angebracht sind.

## Claims

1. A method for manufacturing a medical handle (30) having an illumination device (1, 1', 1"), comprising at least one optical semiconductor component (2), which is designed as a radiation source, a circuit board or multilayer ceramic designed as a carrier (19) for the at least one optical semiconductor component (2) and an electrical power supply unit (41) formed by electric contacts (43) for supplying electric power to the at least one optical semiconductor component (2), wherein the method is **characterized by** the following steps:
• providing a medical handle (30),
• providing the illumination device (1, 1', 1"),
• sheathing the illumination device (1, 1', 1") with a transparent thermoplastic polymer, such that it forms a sheathing (42), which surrounds the at least one optical semiconductor component (2) and the circuit board or the multilayer ceramic on all sides and surrounds the electrical contacts (43) at least partially, and
• attaching the at least one sheathed optical semiconductor component (2) in or on the medical handle (30).

2. The method for manufacturing a medical handle (30) according to Claim 1,
**characterized in that**
the sheathing is performed by providing a solid thermoplastic polymer, liquefying the thermoplastic polymer and curing the thermoplastic polymer.

3. The method for manufacturing a medical handle (30) according to Claim 1 or 2,
**characterized in that**
the sheathing is performed by heating the thermoplastic polymer, preferably to temperatures of approximately max. 310°C, especially preferably to temperatures of approximately max. 280°C.

4. The method for manufacturing a medical handle (30) according to any one of the preceding claims, **characterized in that**
before sheathing it with the thermoplastic polymer, the at least one optical semiconductor component (2) is arranged in a mold and the thermoplastic polymer is introduced into the mold.

5. The method for manufacturing a medical handle (30) according to Claim 4,
**characterized in that**
the thermoplastic polymer is injected into the mold under pressure.

6. The method for manufacturing a medical handle (30) according to any one of the preceding claims, **characterized in that**
the at least one optical semiconductor component (2) is sheathed by the thermoplastic polymer such, that the thermoplastic polymer contacts either at least the radiation-emitting surface (2A) of the at least one optical semiconductor component (2) or components connected to the radiation-emitting surface (2A).

7. The method for manufacturing a medical handle (30) according to any one of the preceding claims, **characterized in that**
the sheathing (42) is manufactured in one piece.

8. A medical handle (30) having an illumination device (1, 1', 1"), comprising at least one optical semiconductor component (2) designed as a radiation source, a circuit board or a multilayer ceramic designed as a carrier (19) for the at least one optical semiconductor component (2) and an electrical power supply unit (41) formed by electric contacts (43) for supplying electric power to the at least one optical semiconductor component (2), **characterized in that**
the illumination device (1, 1', 1") comprises a sheathing (42) with a transparent thermoplastic polymer, which surrounds the at least one optical semiconductor component (2) and the circuit board or the multilayer ceramic on all sides and partially surrounds the electrical contacts (43).

9. The medical handle (30) according to Claim 8, **characterized in that** the at least one optical semiconductor component (2) is sheathed by the thermoplastic polymer such, that the thermoplastic polymer contacts either at least the radiation-emitting surface (2A) of the at least one optical semiconductor component (2) or components connected to the radiation-emitting surface (2A).

10. The medical handle (30) according to Claim 8 or 9, **characterized in that** the sheathing (42) formed by the thermoplastic polymer is designed in one piece.

11. The medical handle (30) according to Claim 8 or 9, **characterized in that** the sheathing formed by the thermoplastic polymer is designed in two or more pieces.

12. The medical handle (30) according to Claim 11, **characterized in that** the sheathing of transparent thermoplastic polymer comprises:
• a base body (3, 3') having a first end (4), a second end (5) and at least one receptacle (8, 8'), wherein the at least one receptacle (8, 8') extends from the second end (5) of the base body (3, 3') in the direction of the first end (4),
• a light-emitting window (9, 9', 9"), which is formed by the part of the base body (3, 3'), which extends from the at least one receptacle (8, 8') up to the first end (4) of the base body (3, 3'),
• a cover device (11, 11'), which is connected to the base body (3, 3') such, that the at least one receptacle (8, 8') forms a hermetically sealed interior space,
wherein the at least one optical semiconductor component (2) is arranged in the hermetically sealed interior space.

13. The medical handle (30) according to Claim 12, **characterized in that** the at least a portion of the cover device (11, 11') is accommodated in the at least one receptacle of the base body (3, 3').

14. The medical handle (30) according to Claim 12 or 13, **characterized in that** the cover device (11, 11') comprises a sealing body (12) having a first end (13) and a second end (14), wherein the sealing body (12) is preferably formed with a borehole (15) extending from the first end (13) to the second end (14) and a wall (16) enclosing the borehole (15).

15. The medical handle (30) according to Claims 8-14, **characterized in that** the sheathing (42) of the illumination device (1, 1') is manufactured from thermoplastic polymer whose re-softening point is more than 120°C.

16. The medical handle (30) according to Claims 8-15, **characterized in that** the electrical contacts (43) of the electrical power supply unit (41) are connected through boreholes (17, 22, 44) in the sheathing (3, 3', 11, 11', 42) to the at least one optical semiconductor component (2).

17. The medical handle (30) according to Claims 8-15, **characterized by** an inductive electrical power supply unit having at least one first coil, which is arranged in the hermetically sealed interior space, and having at least one second coil, which is provided outside of the hermetically sealed interior space, wherein preferably additional electronic components, in particular rectifiers, current regulators, AC-DC converters, capacitors or resistors, are provided between the first coil and the at least one optical semiconductor component.

18. The medical handle (30) according to Claims 8-17, **characterized in that** the circuit board or multilayer ceramic is formed in a ring shape with a central borehole, on which a plurality of optical semiconductor components (2) are mounted.

## Revendications

1. Procédé pour la fabrication d'une pièce à main médicale (30) avec un dispositif d'éclairage (1, 1', 1"), lequel comprend au moins un composant optique à semi-conducteur (2) réalisé en tant que source de rayonnement, une platine ou céramique multicouches réalisée en tant que support (19) pour l'au moins un composant optique à semi-conducteur (2) et un dispositif d'alimentation électrique (41) formé par des contacts électriques (43) pour l'alimentation en courant de l'au moins un composant optique à semi-conducteur (2), dans lequel le procédé se **caractérise par** les étapes suivantes :
• la mise à disposition d'une pièce à main médicale (30),
• la mise à disposition du dispositif d'éclairage (1, 1', 1"),
• l'enveloppement du dispositif d'éclairage (1, 1', 1") avec une matière synthétique thermoplastique transparente de manière à obtenir une enveloppe (42) qui entoure de tous les côtés l'au moins un composant optique à semi-conducteur (2) et la platine ou céramique multicouches et enveloppe partiellement les contacts électriques (43), et
• la fixation de l'au moins un composant optique à semi-conducteur (2) enveloppé dans ou sur la pièce à main médicale (30).

2. Procédé pour la fabrication d'une pièce à main médicale (30) selon la revendication 1, **caractérisé en ce que**
l'enveloppement s'effectue par la mise à disposition d'une matière synthétique thermoplastique dure, la liquéfaction de la matière synthétique thermoplastique et le durcissement de la matière synthétique thermoplastique.

3. Procédé pour la fabrication d'une pièce à main médicale (30) selon la revendication 1 ou 2, **caractérisé en ce que**
l'enveloppement s'effectue par chauffage de la matière synthétique thermoplastique, de préférence à des températures d'environ 310°C au maximum, de manière particulièrement préférée, à des températures d'environ 280°C au maximum.

4. Procédé pour la fabrication d'une pièce à main médicale (30) selon l'une des revendications précédentes, **caractérisé en ce que**
avant l'enveloppement avec la matière synthétique thermoplastique, l'au moins un composant optique à semi-conducteur (2) est disposé dans un moule et **en ce que** la matière synthétique thermoplastique est introduite dans le moule.

5. Procédé pour la fabrication d'une pièce à main médicale (30) selon la revendication 4, **caractérisé en ce que**
la matière synthétique thermoplastique est injectée sous pression dans le moule.

6. Procédé pour la fabrication d'une pièce à main médicale (30) selon l'une des revendications précédentes, **caractérisé en ce que**
l'au moins un composant optique à semi-conducteur (2) est enveloppé par la matière synthétique thermoplastique de telle sorte que la matière synthétique thermoplastique entre en contact soit avec au moins la surface émettant le rayonnement (2A) de l'au moins un composant optique à semi-conducteur (2), soit avec les composants à la suite de la surface émettant le rayonnement (2A).

7. Procédé pour la fabrication d'une pièce à main médicale (30) selon l'une des revendications précédentes, **caractérisé en ce que**
l'enveloppe (42) est fabriquée d'une seule pièce.

8. Pièce à main médicale (30) avec un dispositif d'éclairage (1, 1', 1"), lequel comprend
au moins un composant optique à semi-conducteur (2) réalisé en tant que source de rayonnement, une platine ou céramique multicouches réalisée en tant que support (19) pour l'au moins un composant optique à semi-conducteur (2) et un dispositif d'alimentation électrique (41) formé par des contacts électriques (43) pour l'alimentation en courant de l'au moins un composant optique à semi-conducteur (2), **caractérisée en ce que**
le dispositif d'éclairage (1, 1', 1") présente une enveloppe (42) avec une matière synthétique thermoplastique transparente qui entoure de tous les côtés l'au moins un composant optique à semi-conducteur (2) et la platine ou céramique multicouches, et entoure partiellement les contacts électriques (43).

9. Pièce à main médicale (30) selon la revendication 8, **caractérisée en ce que**
l'au moins un composant optique à semi-conducteur (2) est enveloppé par la matière synthétique thermoplastique de telle sorte que la matière synthétique thermoplastique entre en contact soit avec au moins la surface émettant le rayonnement (2A) de l'au moins un composant optique à semi-conducteur (2), soit avec les composants à la suite de la surface émettant le rayonnement (2A).

10. Pièce à main médicale (30) selon la revendication 8 ou 9, **caractérisée en ce que** l'enveloppe (42) formée par la matière synthétique thermoplastique est réalisée d'une seule pièce.

11. Pièce à main médicale (30) selon la revendication 8 ou 9, **caractérisée en ce que** l'enveloppe formée par la matière synthétique thermoplastique est réalisée en deux ou plusieurs pièces.

12. Pièce à main médicale (30) selon la revendication 11, **caractérisée en ce que** l'enveloppe en matière synthétique thermoplastique transparente comprend :
• un corps de base (3, 3') avec une première extrémité (4), une deuxième extrémité (5) et au moins un logement (8, 8'), dans laquelle l'au moins un logement (8, 8') s'étend de la deuxième extrémité (5) du corps de base (3, 3') en direction de la première extrémité (4),
• une fenêtre d'émission de lumière (9, 9', 9") qui est formée par la partie du corps de base (3, 3') qui s'étend depuis l'au moins un logement (8, 8') jusqu'à la première extrémité (4) du corps de base (3, 3'),
• un dispositif de recouvrement (11, 11') qui est relié au corps de base (3, 3') de telle sorte que l'au moins un logement (8, 8') forme un espace intérieur scellé hermétiquement,
dans lequel l'au moins un composant optique à semi-conducteur (2) est disposé dans l'espace intérieur scellé hermétiquement.

13. Pièce à main médicale (30) selon la revendication 12, **caractérisée en ce que**
au moins une partie du dispositif de recouvrement (11, 11') est réceptionnée dans l'au moins un logement du corps de base (3, 3').

14. Pièce à main médicale (30) selon la revendication 12 ou 13, **caractérisée en ce que** le dispositif de recouvrement (11, 11') comprend un corps de fermeture (12) avec une première extrémité (13) et une deuxième extrémité (14), dans laquelle le corps de fermeture (12) est de préférence réalisé avec une forure (15) allant de la première extrémité (13) à la deuxième extrémité (14) et une paroi (16) enfermant la forure (15).

15. Pièce à main médicale (30) selon l'une des revendications 8-14, **caractérisée en ce que**
l'enveloppe (42) du dispositif d'éclairage (1, 1') est fabriquée en une matière synthétique thermoplastique dont le point de ré-ramollissement se situe au-dessus de 120°C.

16. Pièce à main médicale (30) selon l'une des revendications 8-15, **caractérisée en ce que**
les contacts électriques (43) du dispositif d'alimentation électrique (41) sont reliés grâce à des forures (17, 22, 44) dans l'enveloppe (3, 3', 11, 11', 42) à l'au moins un composant optique à semi-conducteur (2).

17. Pièce à main médicale (30) selon l'une des revendications 8-15, **caractérisée par** un dispositif d'alimentation électrique inductif avec au moins une première bobine qui est disposée dans l'espace intérieur scellé hermétiquement, et avec au moins une deuxième bobine qui est prévue à l'extérieur de l'espace intérieur scellé hermétiquement, dans laquelle on prévoit de préférence, entre la première bobine et l'au moins un composant optique à semi-conducteur, d'autres composants électroniques, en particulier des redresseurs, des rhéostats-régulateurs d'intensité, des convertisseurs CA/CC, des condensateurs ou des résistances.

18. Pièce à main médicale (30) selon l'une des revendications 8-17, **caractérisée en ce que**
la platine ou céramique multicouches est réalisée en forme annulaire avec une forure centrale sur laquelle plusieurs composants optiques à semi-conducteur (2) sont mis en place.
